# EUROPEAN PATENT SPECIFICATION

(11) **EP 4 632 758 B1**
(45) Date of publication and mention of the grant of the patent: **18.11.2026**
(21) Application number: 24315161.0
(22) Date of filing: 12.04.2024
(51) Int. Cl.: G16H 30/40, G16H 50/50, G06T 13/40

(54) **SYSTEM AND METHOD FOR GENERATING PATIENT-SPECIFIC TEMPLATES OF AORTIC LEAFLETS**
SYSTEM UND VERFAHREN ZUR ERZEUGUNG PATIENTENSPEZIFISCHER VORLAGEN FÜR AORTENBLÄTTCHEN
SYSTÈME ET PROCÉDÉ DE GÉNÉRATION DE MODÈLES SPÉCIFIQUES À UN PATIENT DE FEUILLETS AORTIQUES

(43) Date of publication of application: 15.10.2025
(73) Proprietor: Université d'Aix-Marseille, 13007 Marseille (FR); Centre National de la Recherche Scientifique, 75016 Paris (FR); Assistance Publique - Hôpitaux de Marseille, 13005 Marseille (FR); Ecole Centrale de Marseille, 13013 Marseille (FR)
(72) Inventor: Lenoir, Marien, 13005 Marseille (FR); Macé, Loic, 13011 Marseille (FR); Favier, Julien, 13013 Marseille (FR); Cheylan, Isabelle, 13490 Jouques (FR); Fringand, Tom, 13001 Marseille (FR)
(74) Representative: Cornuejols, Marine Sophie

(56) References cited:
- US-B2- 9 788 940
- LABROSSE M R ET AL: "Geometric modeling of functional trileaflet aortic valves: Development and clinical applications", JOURNAL OF BIOMECHANICS, PERGAMON PRESS, NEW YORK, NY, US, vol. 39, no. 14, 1 January 2006 (2006-01-01), pages 2665 - 2672, XP024980382, ISSN: 0021-9290, [retrieved on 20060101], DOI: 10.1016/J.JBIOMECH.2005.08.012
- MACÉ LOÏC GEORGES ET AL: "Three-dimensional modelling of aortic leaflet coaptation and load-bearing surfaces: in silico design of aortic valve neocuspidizations", AIX MARSEILLE UNIV, CNRS, CENTRALE MED, M2P2, MARSEILLE, FRANCE, vol. 39, no. 1, 3 July 2024 (2024-07-03), XP093205420, ISSN: 2753-670X, Retrieved from the Internet <URL:https://academic.oup.com/icvts/article-pdf/39/1/ivae108/58537018/ivae108.pdf> [retrieved on 20240913], DOI: 10.1093/icvts/ivae108
- HOSNY AHMED ET AL: "Pre-procedural fit-testing of TAVR valves using parametric modeling and 3D printing", JOURNAL OF CARDIOVASCULAR COMPUTED TOMOGRAPHY, vol. 13, no. 1, 1 January 2019 (2019-01-01), AMSTERDAM, NL, pages 21 - 30, XP093205431, ISSN: 1934-5925, Retrieved from the Internet <URL:https://pdf.sciencedirectassets.com/274167/1-s2.0-S1934592518X00082/1-s2.0-S1934592518301722/main.pdf?X-Amz-Security-Token=IQoJb3JpZ2luX2VjENv//////////wEaCXVzLWVhc3QtMSJIMEYCIQDPzdQwdV3md+O8KYsdJiSqKaFZEFyy7KSQxhnCboUSUgIhAMLEcgc8ItRiVy6qxY5bngjSaniUrx0GnBcbGS28ABNiKrIFCBQQBRoMMDU5MDAzNTQ2ODY1Igzgb> [retrieved on 20240913], DOI: 10.1016/j.jcct.2018.09.007

## Description

### TECHNICAL FIELD

The present invention is in the medical field, and relates to a system and method for generating patient-specific templates of aortic leaflets for aortic valve neocuspidization surgery.

### BACKGROUND

The morphology and function of the aortic valve is complex. A set of well-defined landmarks, including hinges (i.e. nadir of the sinuses of Valsalva), commissures, leaflet tips, and coronary ostia, describe key anatomic landmarks of the aortic valve. The central anatomical features of the aortic valve are the aortic root and the three aortic leaflets.

The aortic root provides the supporting structures for the leaflets within the left ventricle and the ascending aorta. The aortic root extends from the basal attachments of the leaflets within the left ventricle outflow tract to the sinotubular junction. Geometrically, the aortic root is a cylinder with three wall dilatations referred to as sinuses of the valsava. The leaflets, also called cusps, are attached to the root on semilunar structures within the valvular sinuses. Each leaflet is identified by the following dimensions: length of the free edge, length of attachment of the leaflet to the aortic root and geometric height of the leaflet.

These attachment structures interlink at the level of the sinotubular junction forming three commissures. Along with the nadir of the sinuses of Valsalva which described the virtual basal ring, the commissures are relevant anatomic landmarks and part of various measurements of the aortic valve. The function of the aortic valve is to regulate blood flow between the left ventricle and the aorta.

It hemodynamically separates the aorta and the left ventricle. When the ventricular pressure during systole exceeds that in the ascending aorta, the valve leaflets open. In diastole, when the ventricular pressure decreases to less than the aortic pressure, the valve leaflets close.

Aortic valve disease represents a common valvular disease in developed countries, and has the second highest incidence among congenital valve defects. Although aortic root preserving surgery can be used, and minimally invasive procedures are emerging, the management of patients with aortic valve disease remains challenging.

Aortic valve neocuspidization, also called the Ozaki technique is a surgical procedure to reconstruct a damaged or diseased aortic heart valve. US Patent 9,788,940 B2 from Ozaki describes an instrument of patterning cusp for cardiac valve reconstruction. The procedure entails removing the diseased cusps of the native aortic valve, measuring the intercommissural distance, and shaping new aortic valve cusps from the patient's autologous pericardium using specially designed templates and sewing them in position to the removed leaflets. Today, this method of valve reconstruction is gaining popularity, but the templates and meters needed for it, require economic costs, and are made of plastic, which may cause their fragility.

The physiological reliability of an aortic valve is based on its three-dimensional (3D) geometry. Nowadays, most parts of an aortic root can be modeled through Computed Tomography (CT).

Aortic leaflets can be identified through Multiplanar Reconstruction (MPR) and volume rendering by converting CT data from an imaging modality acquired in a certain plane into another plane.

Quantification of the aortic valve is impaired in conventional methods by the need to translate the 3D data set into a set of 2D planes in order to obtain measurements. This gives potentially erroneous results for the curved anatomy of the basal aorta.

A 3D model derived from CT data enables the non-invasive visualization and quantification of the dynamics of the human aortic root and leaflets in functional and diseased valves.

Document LABROSSE M R ET AL: "Geometric modeling of functional trileaflet aortic valves: Development and clinical applications",JOURNAL OF BIOMECHANICS, PERGAMON PRESS, NEW YORK, NY, US, vol. 39, no. 14, 1 January 2006 (2006-01-01), pages 2665-2672 discloses a method to determine by how much the dimensions of the aortic valve components can vary while still maintaining proper function. Measurements were made on silicone rubber casts of human aortic valves to document the range of dimensional variability encountered in normal adult valves. Analytical equations were written to describe a fully three-dimensional geometric model of a trileaflet valve in both the open and closed positions. A complete set of analytical, numerical and graphical tools was developed to explore a range of component dimensions within functional aortic valves. A list of geometric guidelines was established to ensure safe operation of the valve during the cardiac cycle, with practical safety margins.

However leaflet mesh generation, generally based on the Hounsfield number (HU) and surface rendering, remains difficult due to insufficient spatial resolution of medical imaging.

Therefore, modeling of the aortic valve based on its geometric determinants could aid in the establishment of techniques for neocuspidization.

There is thus a need for a solution that solve or mitigate the aforementioned problems of the prior art solutions. The present invention offers such solution.

### BRIEF SUMMARY OF THE INVENTION

The invention is defined by the appended independent claims. The features and advantages of the concepts may be realized and obtained by means of the components pointed out in the appended claims. These and other features of the described technologies will become more fully apparent from the following description and appended claims.

The present invention offers a method to obtain individualized 3D modeling of aortic leaflets coaptation surface and load-bearing surface using Geometric Morphometrics (GM) applied to geometric determinants of aortic valve.

The method of the invention allows 3D surface modeling of the aortic valve and advantageously provides insights for interpretation of Computed Tomography (CT) scans to in silico design aortic valve neocuspidizations.

The method, by making use of geometric morphometrics using landmarks and semilandmarks located along geometric and surgical determinants of the aortic valve combined with non-Uniform Rational Basis Splines (NURBS), allows for reliable 3D modeling of the aortic leaflet coaptation surface and load-bearing surface areas.

Advantageously, the method of the invention may be operated whatever the shape or the pathology of an aortic valve, could it be a tricuspid one (i.e. normally with 3 leaflets natively), or pathological bicuspid or unicuspid valves.

Advantageously, the method may be used to design aortic valve neocuspidization in silico on a case-by-case basis. The separated 2D flattening of the coaptation surface and of the load-bearing surface of 3D neo-leaflets allows generating patient-specific templates as autologous pericardial cutting guides for aortic valve neocuspidizations.

Several and various applications may benefit from using a 3D modeling of an aortic valve as obtained by the method of the invention. The 3D models could facilitate surgical training related to aortic valve morphology or could make surgical neocuspidizations more intuitive for patient teaching, whether through 3D printing or by stereoscopic anaglyph visualization.

The 3D modeling of the aortic valve as provided by the method of the present invention, using GM applied to geometric determinants, enables a precise definition of the 3D shape of the aortic leaflets. Separate modeling of the coaptation surface and of the load-bearing surface, through the use of commissural coaptation heights and Inferior Lines of Coaptation (ILCs), enables to provide a surface 2D planar projection of the neo-leaflets that allows for in silico experimental study of aortic neocuspidizations.

A first aspect of the invention relates to a computer implemented method for generating patient-specific templates of aortic leaflets. The method comprises the steps of:
- generating a 3D model from digital images of a native aortic root and aortic leaflets of a patient;
- generating 3D neo-leaflets models by simulating in silico aortic leaflet neocuspidization from the 3D model with parameters of developable coaptation surfaces and of non-developable load-bearing surfaces of aortic leaflets;
- realizing a 2D surface flattening of the coaptation surfaces of the 3D neo-leaflets models;
- realizing a 2D surface flattening of the load-bearing surfaces of the 3D neo-leaflets models;
- merging a 2D coaptation surface with a 2D load-bearing surface for each neo-leaflet model; and
- generating a patient-specific template for each aortic leaflet.

In an embodiment, the step of generating a 3D model comprises a first processing step based on a Geometric Morphometrics (GM) approach and a second processing step based on Non-Uniform Rational Basis Splines (NURBS) curves.

In an embodiment, the first processing step based on Geometric Morphometrics comprises:
- using Hounsfield threshold values, segmenting the aortic root by placing top and bottom commissural coaptation landmarks and nadir of each sinus landmarks;
- using Multiplanar reconstruction, placing landmarks corresponding to top and bottom central coaptation points, and placing semilandmarks along Leaflet Free Margins (LFMs) and Inferior Lines of Coaptation (ILCs); and
- using surface rendering, placing semilandmarks along the Leaflet Insertion Line (LIL) on the aortic root.

In an embodiment, the second processing step based on NURBS curves comprises:
- using control point of NURBS curves, drawing each geometric determinant of the leaflets and of the central, the lateral and the commissural coaptation heights;
- using the geometric determinants, modeling a coaptation 3D NURBS surface, and modeling a load-bearing 3D NURBS surface; and
- merging a coaptation 3D NURBS surface with a load-bearing 3D NURBS surface to generate a 3D surface modeling for each aortic leaflet.

In another embodiment, the step of generating 3D neo-leaflets models comprises using native coaptation center in simulation of leaflet extension.

In yet another embodiment, the step of generating 3D neo-leaflets models comprises using centroids of the sinotubular junction and aortic root base in simulation of leaflet resections.

In a further embodiment, the step of realizing a 2D surface flattening of the coaptation surfaces is operated through a standard software control of a Computer-Aided Design tool as the coaptation surfaces are developable surfaces having a curvature in a single direction.

In another embodiment, the step of realizing a 2D surface flattening of the load-bearing surfaces is operated by -a dedicated algorithm as the load-bearing surfaces are non-developable surfaces, comprising the steps of:
- modeling the load-bearing surfaces in the same plane as that for the 2D surface flattening of the coaptation surfaces, by extending the load-bearing surfaces using NURBS lines thereby defining NURBS lines extension;
- setting the length of each NURBS lines extension firstly at value of the geometric height minus the central coaptation height of each neo-leaflet for the median axis of a considered neo-leaflet, and secondly at value of the two complementary radial axes for the intermediate geometrical positions between the median axis, on either side of it, and for the lower edge of the 2D flattened coaptation surface;
- modeling the interpolated neo-LIL curve with the bottom commissural landmarks plus landmarks located at the ends of each NURBS lines extension; and
- constructing 2D load-bearing neo-surfaces as a network of neo-ILC curves, neo-LIL curves, and neo-gH curves.

In a variant, more axes corresponding to each landmark placed on the LIL curves, are used to increase the reliability of the 2D surface flattening of the load-bearing surface.

In another embodiment, the step of generating the 3D model further comprising a step of exporting the 3D model as a mesh file for 3D printing or for 3D stereoscopic visualization.

In yet another embodiment, the method further comprises a step of printing the patient-specific template of each aortic leaflet for use as autologous pericardial cutting guides for aortic valve neocuspidization surgery.

A second aspect of the invention relates to a system for generating patient-specific templates of aortic leaflets, the system comprising means for performing the steps of the method according to anyone of claims.

The present disclosure further includes a computer program product comprising code instructions effective to cause a device to perform the steps of the method discussed above, and throughout this description, when executed by a computer.

Even if the following description presents several examples of embodiments of the method of the invention, these examples are not intended to limit the scope of the invention. The illustrated exemplary embodiments present both the essential characteristics of the invention as well as additional characteristics linked to the embodiments considered.

### BRIEF DESCRIPTION OF THE DRAWINGS

Embodiments of the invention will now be described with reference to the accompanying drawings, in which:
Figure 1 shows general steps for obtaining patient-specific templates of aortic leaflets according to an embodiment of the invention;
Figure 2 shows the two phases of the process of the invention for constructing a patient-specific template using Geometric Morphometrics and NURBS;
Figure 3 shows different steps for generating a 3D model of the aortic root and of the aortic leaflets using Geometric Morphometrics;
Figure 4 illustrates with CT scans different steps of figure 3;
Figure 5 shows different steps for generating 3D models of each neo-leaflet using Non-Uniform Rational Basis Splines;
Figure 6 illustrates an aortic root mesh, landmarks, and 3D NURBS curves for modeling of the leaflets according to different steps of figure 5;
Figure 7a illustrates on Parts A-D 3D surfaces with NURBS curves and on Parts E-F solid modeling of aortic leaflets and aortic root obtained at different steps of figure 5;
Figure 7b illustrates a complete aortic root model plus leaflets with thickness obtained at different steps of figure 5;
Figure 8 shows steps of aortic leaflets neocuspidization according to a first and a second embodiment of the invention;
Figure 9 illustrates aortic leaflets neocuspidization for the embodiments of figure 8;
Figure 10 shows different steps of aortic leaflets neocuspidization for 2D surface flattening according to the embodiments of figure 8;
Figure 11 illustrates pericardial leaflets 2D surface flattening and 2D solid modeling of three neo-leaflets templates according to the invention;
Figure 12 illustrates a gradual increase in gH, ILC and LFM length using 3D NURBS curves.

### DETAILED DESCRIPTION OF THE INVENTION

Various embodiments of the disclosed methods and arrangements are discussed in detail below. While specific implementations are discussed, it should be understood that this is done for illustration purposes only.

Generally speaking, the method herein described aims to model the coaptation and load-bearing surface areas of aortic leaflets and adapt this workflow to 3D modeling and 2D flattening design of aortic valve neocuspidizations. Geometric morphometrics, using landmarks and semilandmarks, are applied to the geometric determinants of the aortic leaflets from computed tomography data under mid-diastolic loading conditions, followed by an isogeometric analysis using Non-Uniform Rational Basis Splines (NURBS). The geometric determinants of leaflet geometry are defined as 3D NURBS curves, and the coaptation surfaces and the load-bearing surfaces of the leaflet are defined as 3D NURBS surfaces. 3D neocuspidizations are obtained either by shifting the upper central coaptation landmark toward the sinotubular junction or by using parametric neo-landmarks placed on a centerline drawn between the centroid of the aortic root base and the centroid of a circle circumscribing the three upper commissural landmarks. 2D flattening of the previous 3D model, using this new algorithm, allows obtaining patient-specific templates of the three autologous pericardial neo-leaflets, enabling to reliably define the coaptation and load-bearing surfaces of the aortic neo-leaflets and enabling to anticipate an improvement in haemodynamics, a source of sustainability in the surgical management of the aortic valve. Use of these templates, printed before the surgery, also aims to decrease the aortic cross-clamping time during cardiac procedure.

Before detailing the method of the present invention, the following concepts related to landmarks, semilandmarks and Non-Uniform Rational Basis Splines (NURBS) are defined.

As shown on Table 1, landmarks have been defined as type I, type II, and so-called semilandmarks type III. Landmarks can be exported with an aortic root mesh (i.e., a blood pool isosurface of a left ventricular outflow tract, interleaflet triangles, sinuses of Valsalva, and aorta) to a Computer-Aided Design (CAD) tool to construct NURBS as shown on Table 2.

**Table 1: Landmark-based Geometric Morphometrics (GM)**

| Landmark | Definition |
|---|---|
| Type I: Anatomic | A point corresponding to a clearly defined location, such as the top of the commissures, or a point where two structures meet, such as the central coaptation points |
| Type II: Geometric | A point indicated by a location that can be identified in all cases, such as the nadir of the sinuses of Valsalva (i.e., corresponding to a maximum of curvature) |
| Type III: Semilandmarks | Points allowing the capture of relative geometric shapes along curves or anatomical ridges Applies to the leaflet insertion line, geometric height, leaflet free margin, and inferior line of coaptation |

**Table 2: Non-Uniform Rational Basis Splines (NURBS) lines, curves, and surfaces**

| NURBS type | Definition | Applies to |
|---|---|---|
| Line/polyline (degree 1) | A line/polyline is drawn between type I or II different landmarks. | Central, lateral and commissural coaptation lines; |
| | | Leaflet free margin (neocuspidization); gH (neocuspidization) |
| ControlPoint curve (degree 3) | A ControlPoint curve is starting and ending at type I or II different landmarks. | Leaflet insertion line; * |
| | | Geometric height; ** |
| | | Leaflet free margin; |
| | Semilandmarks act as CP and determine the shape of a curve. | Inferior line of coaptation |
| | The curve does not necessarily pass through each CP, but each CP influences the curve's shape. | |
| Interpolated curve (degree 3) | A curve that passes exactly through each landmark. | Parametric Inferior line of coaptation (for neocuspidization); |
| | Interpolated curves are useful when the modeling has specific anatomic or geometric constraints for where the curve must pass | |
| | | Leaflet insertion line (2D flattening during neocuspidization) |
| Surface from a network of curves | A surface resulting from a network of crossing lines, interpolated and/or CP curves. | Coaptation surfaces; |
| | | Load-bearing surface areas; |
| | | Neocuspidization surface areas |

| | | |
|---|---|---|
| * The length of the leaflet insertion line incorporates two corresponding commissural coaptation heights. ** Length of each geometric height incorporates the central coaptation height. | | |

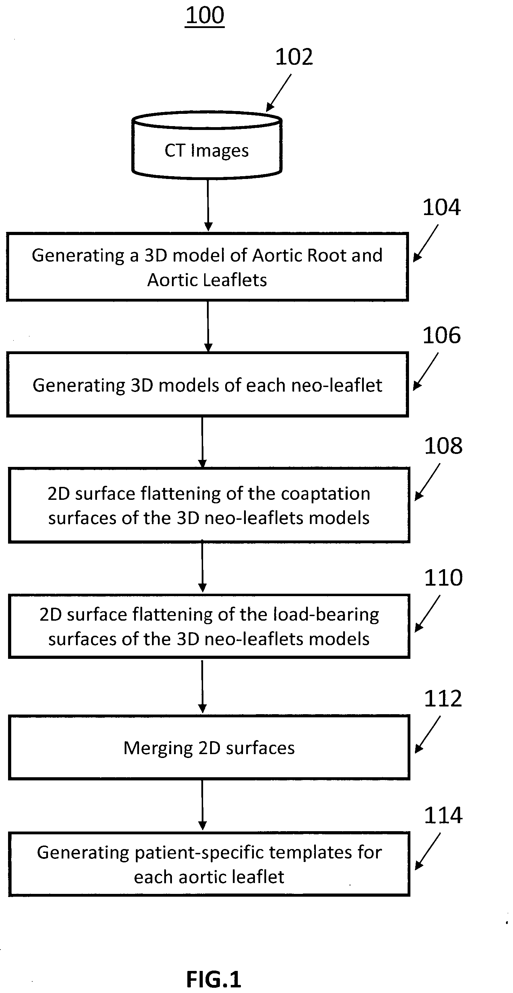

Going first to figure 1, a general flowchart of a process 100 for obtaining patient-specific templates of aortic leaflets according to an embodiment of the invention is described.

The embodiment is described for an aortic valve of tricuspid normal shape, having three leaflets natively. However, the method may be operated for different shapes of aortic valves and different pathologies, such as bicuspid aortic valve having two leaflets, or unicuspid aortic valves having one leaflet.

**In** the latter case, a specific re-localization of landmarks is computed with a computer-aided design tool, in particular, landmarks that are commissural ones are placed using geometric (parametric) values of healthy tricuspid valves, enabling the creation of tricuspid neocuspidization as in a healthy valve and whatever the initial pathology. So, even if the valve is bicuspid (bi-commissural) or unicuspid (uni-commissural), advantageously three templates may be generated as this is the more relevant from a hemodynamic point of view.

A patient-specific template may be obtained through a first phase (steps 104, 106) for 3D models generation with landmarks, and a second phase (steps 108, 110, 112) for 2D models projection of the 3D models.

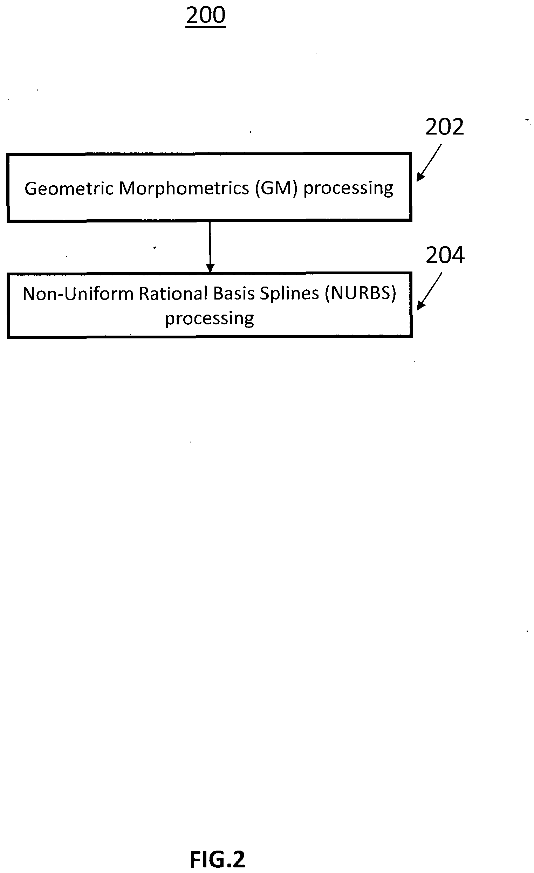

As shown on figure 2, the first phase 200 comprises a first processing 202 using geometric morphometrics followed by a second processing 204 using Non-Uniform Rational Basis Splines.

The process 100 begins with a selection of several Computed Tomography images 102 of a patient. The CT scans available as DICOM files (meaning Digital Imaging and Communications in Medicine, a technical standard for the digital storage and transmission of medical images) may be imported into a viewer for the selection. The CT scans of patients are preferably chosen based on the quality of their contrast and electrocardiogram (EKG) synchronization.

On a first step 104, the process allows generating a 3D model of the aortic root and of the aortic leaflets from the selected CT images of the patient.

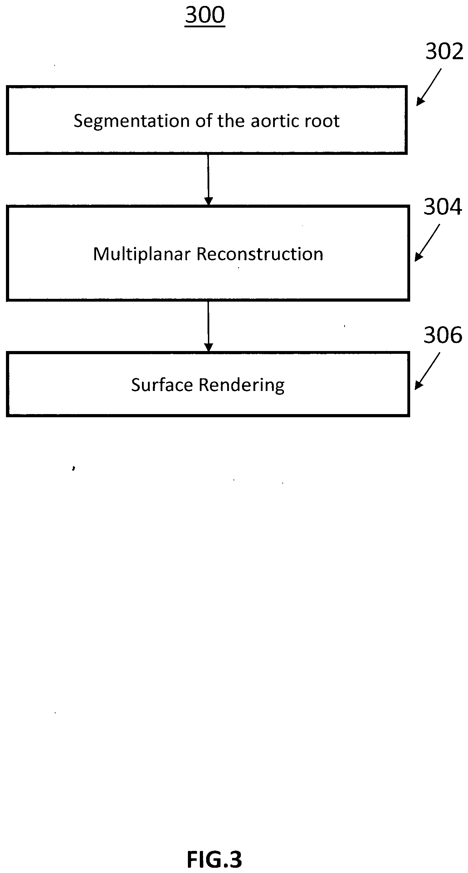

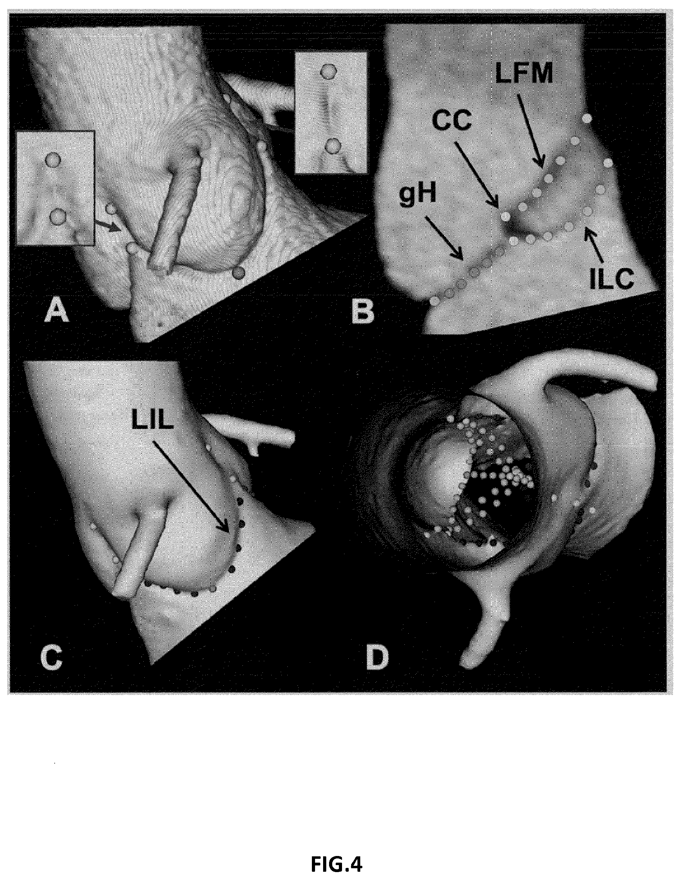

In an embodiment shown on figure 3 and illustrated by figure 4, the aortic root is first segmented 302 using Hounsfield threshold values. Type I (i.e. top and bottom commissural coaptation landmarks) and type II (i.e. nadir of each sinus) landmarks are placed on an initial template as shown on part A of figure 4.

Then in a second step 304 of a Multiplanar Reconstruction (MPR) using a bisecting center plane orthogonal to the virtual basal ring (i.e. plane passing through the three nadirs), type I landmarks corresponding to the top and bottom central coaptation (CC) points are positioned. Additionally, semilandmarks are placed along the Leaflet Free Margins (LFMs) and the Inferior Lines of Coaptation (ILCs). The LFMs and ILCs are the superior and inferior curves of coaptation surfaces, respectively. An ILC is also known as the junction line of a leaflet or a caudal limit of coaptation. Next, semilandmarks are placed along the geometric Height (gH) curves, except for the central coaptation height, passing through the corresponding nadir. Part B of figure 4 illustrates the placement of the LFMs, ILCs, gH and shows the CC.

It is to be noted that the gH curves and the coaptation surfaces are not always located in the same bisecting plane due to the frequent asymmetry of the leaflets.

In an embodiment, additional semilandmarks in the radial directions are placed during this step 304 for a later use during 2D surface flattening.

In a further step 306, semilandmarks are placed along the leaflet insertion line (LIL) on the aortic root, except for the commissural coaptation height, using surface rendering. Part C of figure 4 illustrates this placement.

Part D of figure 4 illustrates a complete initial template including the landmarks, the semilandmarks, and the aortic root as a triangle-based mesh. This template may be exported as a mesh file for pursuing the process 100 with step 106.

Step 106 allows the process to generate 3D models of each neo-leaflet using Non-Uniform Rational Basis Splines.

The mesh files are imported into a CAD software tool configured to use NURBS to create 3D mathematical representations from landmarks and semilandmarks defined as control points.

As known, control points determine the shape of a curve. Typically, each point of the curve is computed by taking a weighted sum of a number of control points. The weight of each point varies according to the governing parameter. For a curve of degree d, the weight of any control point is only nonzero in d+1 intervals of the parameter space. Within those intervals, the weight changes according to a polynomial function (basis functions) of degree d. At the boundaries of the intervals, the basis functions go smoothly to zero, the smoothness being determined by the degree of the polynomial. Adding more control points allows better approximation to a given curve, although only a certain class of curves can be represented exactly with a finite number of control points. NURBS curves also feature a scalar weight for each control point. This allows for more control over the shape of the curve without unduly raising the number of control points. In particular, it adds conic sections like circles and ellipses to the set of curves that can be represented exactly. The term rational in NURBS refers to these weights.

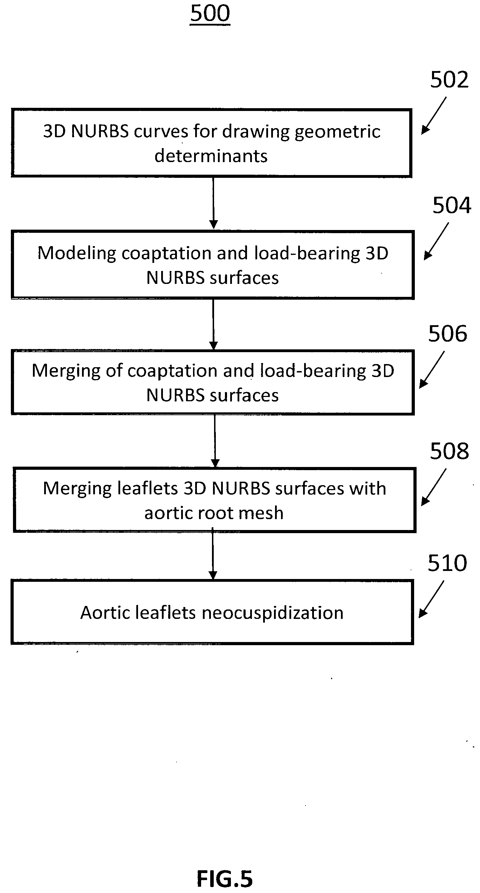

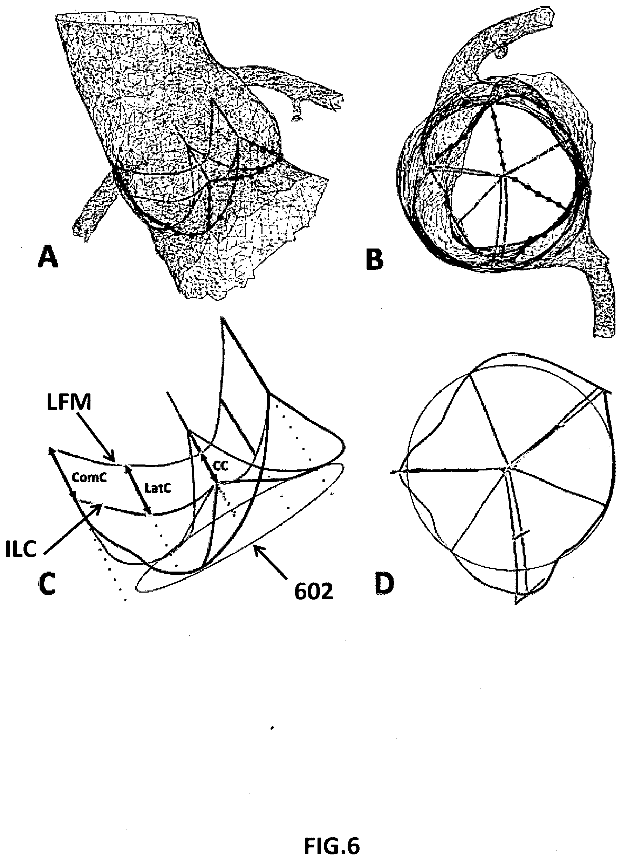

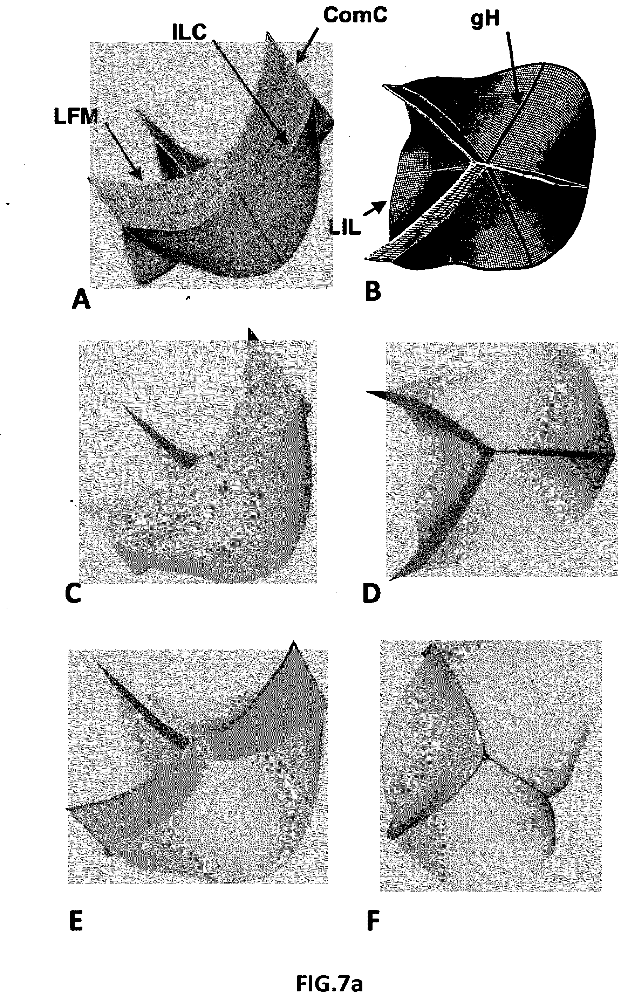

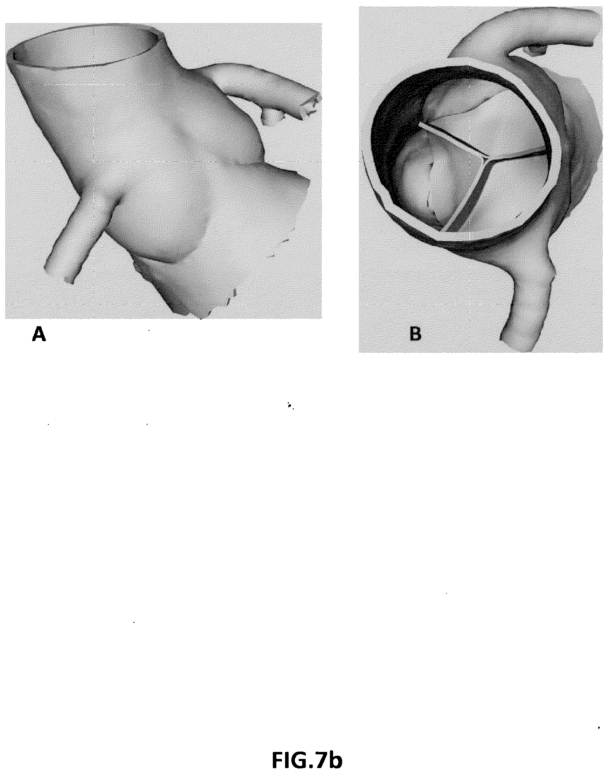

In an embodiment shown on figure 5 and illustrated by figures 6 and 7, on a first step 502, control point NURBS curves (or interpolated control point as appropriate) are used to draw each geometric determinant of the leaflets, as well as the central, lateral, and commissural coaptation heights. The lateral coaptation heights are measured using a line drawn between the middle of the LFM and the middle of the ILC, limited to the commissure under study.

For sake of illustration, parts A and B of figure 6 illustrate the landmarks, semilandmarks, and aortic root mesh, parts C and D illustrate the NURBS lines and curves. The black double-headed arrows represent the central, lateral and commissural coaptation lines (CC, LatC, ComC). It is to be noted that nadirs are not always at the middle of the leaflet insertion line as illustrated in this case. The black circle 602 illustrates the virtual basal ring. The dotted lines illustrate the distance between the different coaptation heights and the plane of the aortic root base.

It is to be appreciated that the gH measurements include the central coaptation height, whereas LIL measurements include the two ipsilateral commissural coaptation heights.

Going back to figure 5, the process continues with a step 504 wherein the coaptation surface area and the load-bearing surface area are independently modeled from a network of NURBS curves, illustrated on parts A and B of figure 7. The coaptation surfaces comprise a network of four NURBS curves, namely the LFM, the ILC, and the two commissural coaptations ComC lines. The load-bearing surfaces comprise a network of three NURBS curves, namely the ILC, the gH, and the LIL (without the ipsilateral coaptation heights).

On a next step 506, two surfaces of coaptation and of load-bearing are merged to create a single surface for each leaflet, as illustrated on parts C and D of figure 7.

On a next step 508, the process allows joining the NURBS surfaces of the leaflets with the aortic root mesh to constitute the 3D model.

After thickening the aortic root and the leaflets as illustrated on figure 7b, the 3D model may be exported as a mesh file for 3D solid modeling and for any form of 3D stereoscopic anaglyph visualization requiring anaglyph glasses.

The ability to export landmarks with the aortic root mesh is an essential part given that the mesh generation of aortic leaflets is usually incomplete, although they are discernible in MPR. The use of commissural coaptation heights and ILCs increase the accuracy of the 3D modeling by separately modeling the coaptation and the load-bearing surfaces with a physiological diastolic pressure.

This individualized 3D modeling respects the geometry of the aortic valve in terms of the elliptical shape of the aortic root base with respect to the circular aspect of the sinotubular junction, asymmetry of the leaflets, and tilt of the aortic valve.

It is thus essential to consider the three coaptation heights (central, lateral, and commissural) and the ILCs when separately modeling the coaptation surfaces and load-bearing surfaces.

In a next step 510, the process allows an in silico aortic leaflets neocuspidization simulation. The principle of the neocuspidization of the aortic valve is to raise the autologous pericardial neo-LFM to the level of the sinotubular junction (shown as circle 602 circumscribing the three top commissural landmarks).

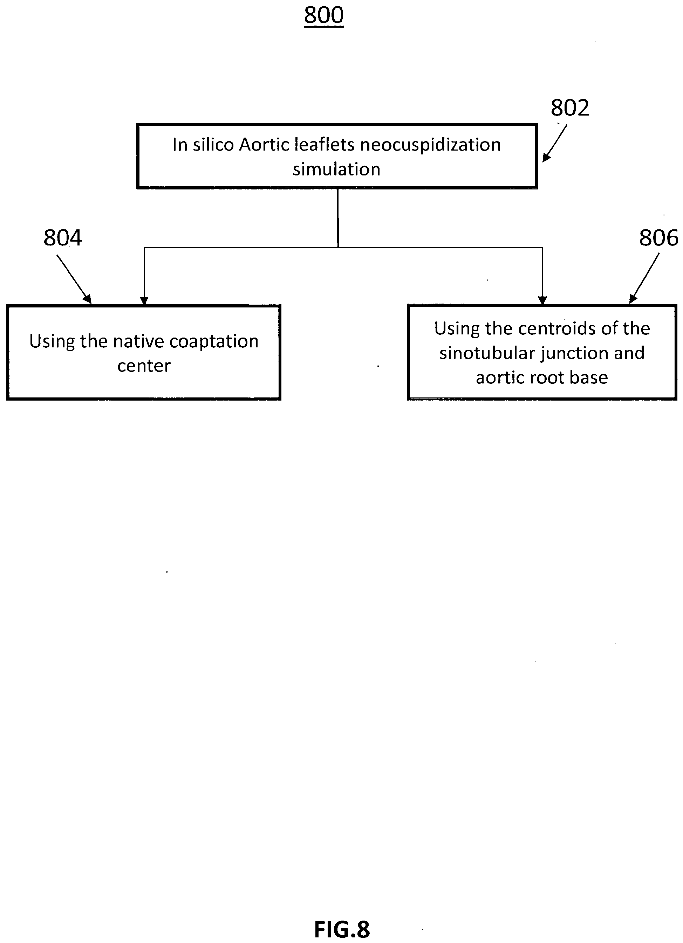

The in silico aortic leaflets neocuspidization simulation 802 may be operated according to two different methods, as shown on figure 8.

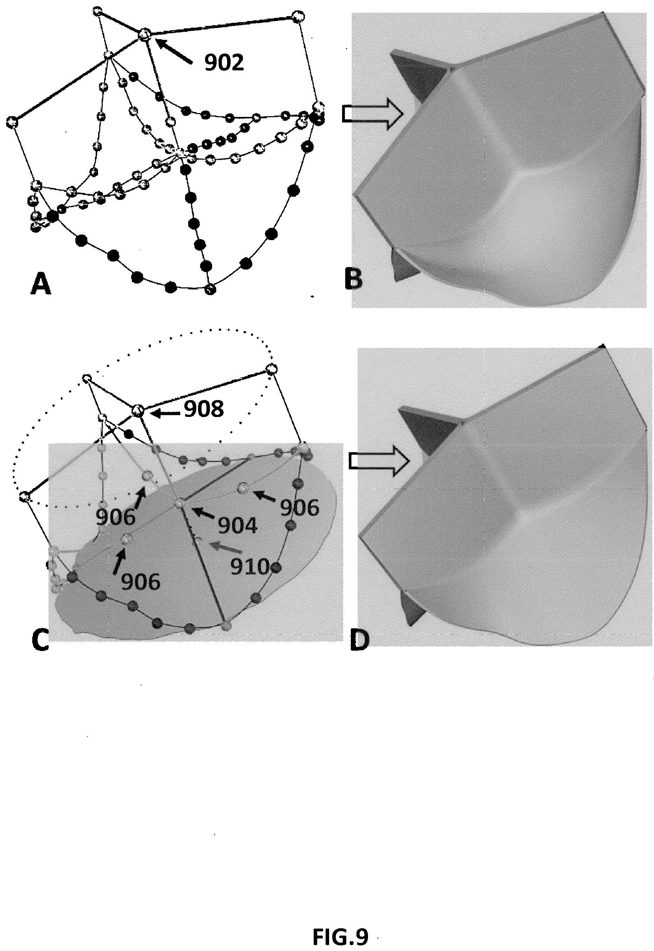

In an embodiment 804 and illustrated on parts A and B of figure 9, the native coaptation center is used for simulating leaflet extension. From the previously obtained 3D model, landmarks corresponding to the LFMs are removed. The load-bearing surfaces remain unchanged. The top central coaptation landmark is transferred to the plane of the sinotubular junction along an extension of the central coaptation line. Neo-LFMs are redrawn as straight lines between the top commissural landmarks and the top central coaptation neo-landmark 902. Part B illustrates neo-leaflets with different LFM lengths.

In another embodiment 806 and illustrated on parts C and D of figure 9, the centroids of the sinotubular junction and aortic root base are used for the simulation. This approach is taken to simulate neocuspidization after leaflet resections. The parametric values are defined as the mean values of previously measured healthy aortic valves. The new central coaptation line connects the centroid of the sinotubular junction (centroid of a circle circumscribing the three upper commissural landmarks) and the centroid of the aortic root base.

As the shape of the aortic root base is elliptical, the centroid of the elliptical surface of the left ventricular outflow tract corresponding to the same plane as the virtual basal ring, is used. The height of the bottom central coaptation neo-landmark 904 on this central axis is parametric. Parametric heights of the bottom lateral coaptation neo-landmarks 906, relative to the aortic root base, are projected onto a line passing, for each commissure, through both the middle of the neo-LFM and the middle of a straight line drawn between the bottom commissural landmark and the bottom central coaptation neo-landmark.

The bottom coaptation neo-landmarks (commissural, lateral, and central) are interpolated to obtain the ILC neo-curves. The LFM neo-curves are described, for each neo-leaflet, as polylines between the top ipsilateral commissural landmarks and the neo-landmark located at the centroid 908 of the sinotubular junction. Neo-gH curves are modeled as straight lines between the nadir of each sinus and the bottom neo-landmark of the central coaptation given the assumption that load-bearing surfaces are curved in only one direction. Surfaces were modeled as described earlier. Part D of figure 9 illustrates a 3D model of neo-leaflets with identical LFM lengths.

Advantageously by using the centroid 908 of a circle circumscribing the three superior commissural landmarks, this embodiment allows obtaining three neo-LFMs of equal length, regardless of the asymmetry of the commissural orientation of the tricuspid aortic valve. In various embodiments, different centers may also be used: (1) the centroid of a triangle defined by the three commissures, (2) the Torricelli point to obtain three leaflets of the same angular sector, and (3) the centroid of the plane corresponding to the sinotubular junction.

Still advantageously as regard to the aortic root base, by using the centroid 910 of its surface allows to consider its elliptical shape.

In alternate embodiments, the centroid of the deepest point of each intervalvular triangles, or the centroid of a closed NURBS curve incorporating the three nadirs and three deepest points of each intervalvular triangles as control points, may be used.

3D solid modeling is the gateway to several applications for training, teaching, and clinical purposes. Inexpensive anaglyph stereoscopic visualization improves depth perception. The 3D printing of the aortic leaflets can be used to obtain pericardial leaflets in 3D or 2D projection. 3D surface modeling accuracy may be evaluated for the load-bearing surfaces of the leaflets. Anaglyph 3D stereoscopic visualization allows assessing the distance of additional landmarks from the load-bearing surfaces and for the aortic root.

Going back to figure 1, the process continues with the second phase for a 2D model projection of the 3D neo-models previously obtained, realizing a 2D surface flattening of the coaptation surfaces of the 3D neo-leaflets models on step 108, and separately realizing 2D surface flattening of the load-bearing surfaces of the 3D neo-leaflets models on step 110.

In an embodiment, step 108 is operated through a standard software control of a Computer-Aided Design tool as the coaptation surfaces are developable surfaces having a curvature in a single direction.

Indeed, when using curvature analysis, the leaflets include a developable surface (the coaptation surface) and an essentially non-developable surface (the load-bearing surface).

Although the load-bearing surfaces may generally be considered as being developable, they are often too complex to flatten into 2D patterns.

Furthermore, the 2D projection of an entire merged leaflet (i.e. a complex double-curved surface) cannot be performed using the 3D model due to a compound curvature.

To face this problem, the inventors have developed a dedicated approach to realize a 2D projection of the load-bearing surface of leaflets in step 110.

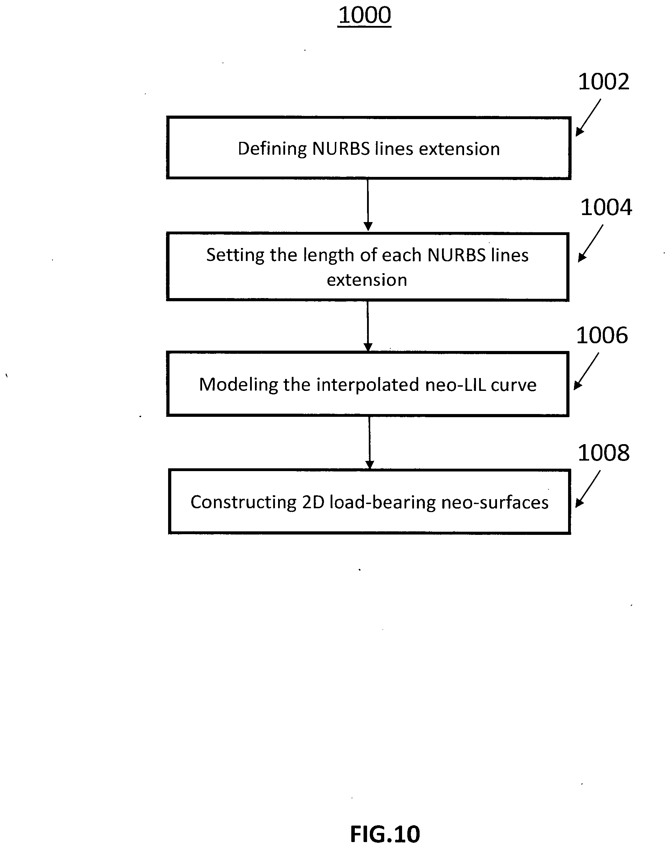

In an embodiment shown on figure 10, a specific algorithm to realize a 2D projection of the load-bearing surface of leaflets follows the steps of:
1002: modeling the load-bearing surfaces in the same plane as that for the 2D f the coaptation surfaces, by extending the load-bearing surfaces using NURBS lines thereby defining NURBS lines extension;
1004: setting the length of each NURBS lines extension firstly at value of the geometric height minus the central coaptation height of each neo-leaflet for the median axis of a considered neo-leaflet, and secondly at value of the two complementary radial axes for the intermediate geometrical positions between the median axis, on either side of it, and for the lower edge of the 2D flattened coaptation surface;
1006: modeling the interpolated neo-LIL curve with the bottom commissural landmarks plus landmarks located at the ends of each NURBS lines extension; and. 1008: constructing 2D load-bearing neo-surfaces as a network of neo-ILC curves, neo-LIL curves, and neo-gH curves.

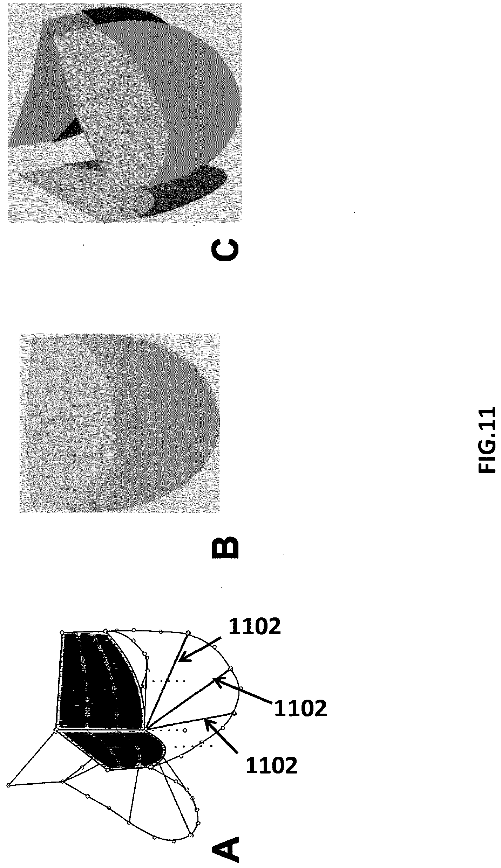

Figure 11 illustrates the possibilities of adjusting, adapting leaflet dimensions to always create an additional margin to be used for suturing neo-cusps in the aortic root.

Part A of figure 11 illustrates a 3D template in a CAD tool with the gH and radial additional axes 1102.

Part B of figure 11 illustrates a 2D flattening of a coaptation surface merged with a neo-leaflet load-bearing 2D surface.

Part C of figure 11 illustrates a 2D solid modeling of three neo-leaflets templates which constitute patient-specific 3D printed pericardial cutting guides.

Going back to figure 1, the process continues with a step 112 of merging the neo-coaptation surfaces and the neo-load-bearing surfaces. After the merging of the two surfaces, a patient-specific template is generated 114 and available for each neo-leaflet.

The patient-specific templates can be used as 3D printed autologous pericardial cutting guides for aortic valve neocuspidization surgery.

Variations in the radial (gH) or circumferential (LFM & ILC) directions make it possible to test and prevent potential failure of the central coaptation thanks to preoperative fluid-structure interaction simulations, and allow to:
- assess different configurations of leaflet hyperelasticity and anisotropy;
- assess dimensional variations of the neo-leaflet from physiologically loaded state (computed tomography data) to the unpressurized state (application of the template onto the autologous pericardium during the cardiac surgery procedure);
- assess the location of the free edge of the neo-leaflets in relation to the position of the coronary arteries in the sinuses of Valsalva, both to assess post-operative perfusion and to subsequently perform TAVR without interfering with coronary perfusion by initially reducing the gH of the neo-leaflets if necessary;
- obtain a suture margin around the template opposite the LIL and the commissural coaptation height.

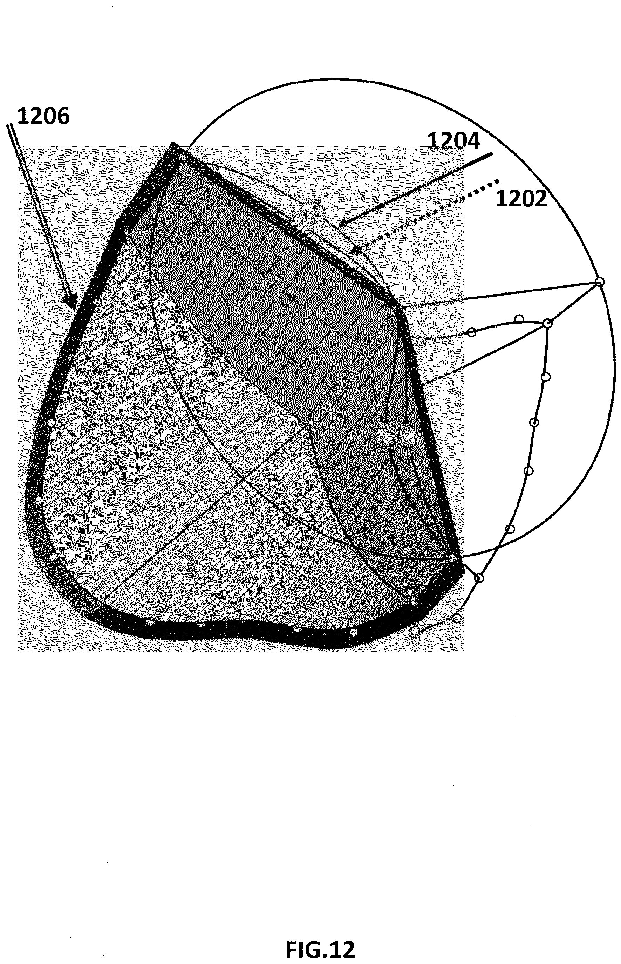

Figure 12 is an illustration of a gradual increase in gH, ILC and LFM length using 3D NURBS curves, with an increase in LFM length from 2.5% (dotted line arrow 1202) to 7% (solid line arrow 1204).

The increase can be used up to more than 20%, which will result in the classical "triscale" aspect of the aortic valve neocuspidization if such three neo-leaflets are included in the native aortic root (grey dots indicate additional control points to theoretically increase the LFM).

The resulting coaptation and load-bearing 3D NURBS surfaces demonstrate in this example, a 7% isotropic increase (double-lined arrow 1206 pointing the dark shaded area).

This process also makes it possible to model and incorporate the suture margin of a neo-leaflet into the 3D printed individualized templates.

The 3D modeling of the aortic valve as per the present invention, using GM applied to geometric determinants of the aortic valve, enables a precise definition of the 3D shape of the aortic leaflets. Separate modeling of the coaptation and load-bearing surfaces, through the additional use of commissural coaptation heights and ILCs, provides a surface 2D planar projection of the neo-leaflets that allows 3D printing of templates for aortic neocuspidizations. These patient-specific templates can be used as 3D printed autologous pericardial cutting guides for aortic valve neocuspidization surgery.

The method may be implemented by a computer system comprising means configured for generating patient-specific templates of aortic leaflets according to the steps of the method. The computer system may comprise at least a processor, a memory and storage operationally in communication. The processor may store a set of non-transitory instructions that when executed may cause the processor to perform or execute any one or more of the aspects and/or methodologies of the present disclosure. The computer system may be coupled to a 3D printer for allowing 3D printing of the templates.

The present description illustrates one embodiment of the invention, but is not limiting. The example is chosen to allow a good understanding of the principles of the invention, and some specific applications, but this is not exhaustive. Nevertheless, the description should allow a person skilled in the art to provide modifications and implementation variants while keeping the same principles.

## Claims

1. A computer-implemented method (100) for generating patient-specific templates of aortic leaflets comprising:
- generating (104) a 3D model from digital images (102) of a native aortic root and aortic leaflets of a patient;
- generating (106) 3D neo-leaflets models by simulating in silico aortic leaflet neocuspidization from the 3D model with parameters of developable coaptation surfaces and of non-developable load-bearing surfaces of aortic leaflets;
- realizing (108) a 2D surface flattening of the coaptation surfaces of the 3D neo-leaflets models;
- realizing (110) a 2D surface flattening of the load-bearing surfaces of the 3D neo-leaflets models;
- merging (112) a 2D coaptation surface with a 2D load-bearing surface for each neo-leaflet model; and
- generating (114) a patient-specific template for each aortic leaflet.

2. The method of claim 1 wherein the step (104) of generating a 3D model comprises a first processing step (202) based on a Geometric Morphometrics 'GM' approach and a second processing step (204) based on Non-Uniform Rational Basis Splines 'NURBS' curves.

3. The method of claim 2 wherein the first processing step (202) based on Geometric Morphometrics comprises:
- using Hounsfield threshold values, segmenting (302) the aortic root by placing top and bottom commissural coaptation landmarks and nadir of each sinus landmarks;
- using Multiplanar reconstruction, placing (304) landmarks corresponding to top and bottom central coaptation points, and placing semilandmarks along Leaflet Free Margins (LFMs) and Inferior Lines of Coaptation (ILCs); and
- using surface rendering, placing (306) semilandmarks along the Leaflet Insertion Line (LIL) on the aortic root.

4. The method of claim 2 or 3 wherein the second processing step (204) based on NURBS curves comprises:
- using control point of NURBS curves, drawing (502) each geometric determinant of the leaflets and of the central, the lateral and the commissural coaptation heights;
- using the geometric determinants, modeling (504) a coaptation 3D NURBS surface, and modeling a load-bearing 3D NURBS surface; and
- merging (506) a coaptation 3D NURBS surface with a load-bearing 3D NURBS surface to generate a 3D surface modeling for each aortic leaflet.

5. The method of anyone of claims 1 to 4 wherein the step (106) of generating 3D neo-leaflets models comprises (804) using native coaptation center in simulation of leaflet extension (804).

6. The method of anyone of claims 1 to 4 wherein the step (106) of generating 3D neo-leaflets models comprises (806) using centroids of the sinotubular junction and aortic root base in simulation of leaflet resections.

7. The method of anyone of claims 1 to 6 wherein the step (108) of realizing a 2D surface flattening of the coaptation surfaces is operated through a standard software control of a Computer-Aided Design tool as the coaptation surfaces are developable surfaces having a curvature in a single direction.

8. The method of anyone of claims 1 to 7 wherein the step (110) of realizing a 2D surface flattening of the load-bearing surfaces is operated by a dedicated algorithm as the load-bearing surfaces are non-developable surfaces, comprising the steps of:
- modeling (1002) the load-bearing surfaces in the same plane as that for the 2D surface flattening of the coaptation surfaces, by extending the load-bearing surfaces using NURBS lines thereby. defining NURBS lines extension;
- setting (1004) the length of each NURBS lines extension firstly at value of the geometric height minus the central coaptation height of each neo-leaflet for the median axis of a considered neo-leaflet, and secondly at value of the two complementary radial axes for the intermediate geometrical positions between the median axis, on either side of it, and for the lower edge of the 2D flattened coaptation surface;
- modeling (1006) the interpolated neo-LIL curve with the bottom commissural landmarks plus landmarks located at the ends of each NURBS lines extension; and
- constructing (1008) 2D load-bearing neo-surfaces as a network of neo-ILC curves, neo-LIL curves, and neo-gH curves.

9. The method of claim 8 wherein more axes corresponding to each landmark placed on the LIL curves, are used to increase the reliability of the 2D surface flattening of the load-bearing surface.

10. The method of anyone of claims 1 to 9 wherein the step (114) of generating the 3D model further comprising a step of exporting the 3D model as a mesh file for 3D printing or for 3D stereoscopic visualization.

11. The method of anyone of claims 1 to 10 further comprising a step of printing the patient-specific template of each aortic leaflet for use as autologous pericardial cutting guides for aortic valve neocuspidization surgery.

12. A system for generating patient-specific templates of aortic leaflets comprising means for performing the steps of the method according to anyone of claims 1 to 11.

13. A computer program product, said computer program product comprising code instructions for performing, when executed by a computer, the steps of the method according to anyone of claims 1 to 11.

## Patentansprüche

1. Computerimplementiertes Verfahren (100) zum Erzeugen von patientenspezifischen Schablonen von Aortenklappensegeln, umfassend:
- Erzeugen (104) eines 3D-Modells aus digitalen Bildern (102) einer nativen Aortenwurzel und von Aortenklappensegeln eines Patienten;
- Erzeugen (106) von 3D-Neo-Klappensegel-Modellen durch In-silico-Simulieren einer Aortenklappensegel-Neokuspidisierung aus dem 3D-Modell mit Parametern von abwickelbaren Koaptationsflächen und von nicht abwickelbaren lasttragenden Flächen von Aortenklappensegeln;
- Durchführen (108) einer 2D-Verflachung der Koaptationsflächen der 3D-Neo-Klappensegel-Modelle;
- Durchführen (110) einer 2D-Verflachung der lasttragenden Flächen der 3D-Neo-Klappensegel-Modelle;
- Zusammenführen (112) einer 2D-Koaptationsfläche mit einer lasttragenden 2D-Fläche für jedes Neo-Klappensegel-Modell; und
- Erzeugen (114) einer patientenspezifischen Schablone für jedes Aortenklappensegel.

2. Verfahren nach Anspruch 1, wobei der Schritt (104) des Erzeugens eines 3D-Modells einen ersten Verarbeitungsschritt (202) auf Grundlage eines Ansatzes der geometrischen Morphometrie "GM" und einen zweiten Verarbeitungsschritt (204) auf Grundlage von Kurven nicht-uniformer rationaler Basis-Splines "NURBS" umfasst.

3. Verfahren nach Anspruch 2, wobei der erste Verarbeitungsschritt (202) auf Grundlage von geometrischer Morphometrie umfasst:
- unter Verwendung von Hounsfield-Schwellenwerten, Segmentieren (302) der Aortenwurzel durch Platzieren von oberen und unteren kommissuralen Koaptationslandmarken und von Landmarken am Nadir jedes Sinus;
- unter Verwendung von multiplanarer Rekonstruktion, Platzieren (304) von Landmarken, die oberen und unteren zentralen Koaptationspunkten entsprechen, und Platzieren von Semilandmarken entlang von freien Klappensegelrändern (LFMs) und unteren Koaptationslinien (ILCs); und
- unter Verwendung einer Oberflächendarstellung, Platzieren (306) von Semilandmarken entlang der Klappensegelinsertionslinie (LIL) an der Aortenwurzel.

4. Verfahren nach Anspruch 2 oder 3, wobei der zweite Verarbeitungsschritt (204) auf Grundlage von NURBS-Kurven umfasst:
- unter Verwendung eines Kontrollpunkts von NURBS-Kurven, Zeichnen (502) jeder geometrischen Determinante der Klappensegel und der zentralen, der lateralen und der kommissuralen Koaptationshöhe;
- unter Verwendung der geometrischen Determinanten, Modellieren (504) einer 3D-NURBS-Koaptationsfläche und Modellieren einer lasttragenden 3D-NURBS-Fläche; und
- Zusammenführen (506) einer 3D-NURBS-Koaptationsfläche mit einer lasttragenden 3D-NURBS-Fläche, um eine 3D-Flächenmodellierung für jedes Aortenklappensegel zu erzeugen.

5. Verfahren nach einem der Ansprüche 1 bis 4, wobei der Schritt (106) des Erzeugens von 3D-Neo-Klappensegel-Modellen (804) ein Verwenden eines nativen Koaptationszentrums bei der Simulation einer Klappensegelverlängerung (804) umfasst.

6. Verfahren nach einem der Ansprüche 1 bis 4, wobei der Schritt (106) des Erzeugens von 3D-Neo-Klappensegel-Modellen (806) ein Verwenden von Schwerpunkten des sinotubulären Übergangs und der Aortenwurzelbasis bei der Simulation von Klappensegelresektionen umfasst.

7. Verfahren nach einem der Ansprüche 1 bis 6, wobei der Schritt (108) des Durchführens einer 2D-Verflachung der Koaptationsflächen über eine standardmäßige Softwaresteuerung eines Werkzeugs für rechnergestützte Konstruktion erfolgt, da die Koaptationsflächen abwickelbare Flächen mit einer Krümmung in einer einzigen Richtung sind.

8. Verfahren nach einem der Ansprüche 1 bis 7, wobei der Schritt (110) des Durchführens einer 2D-Verflachung der lasttragenden Flächen durch einen dedizierten Algorithmus erfolgt, da die lasttragenden Flächen nicht abwickelbare Flächen sind, und folgende Schritte umfasst:
- Modellieren (1002) der lasttragenden Flächen in derselben Ebene wie diejenige für die 2D-Verflachung der Koaptationsflächen durch Erweitern der lasttragenden Flächen unter Verwendung von NURBS-Linien, wodurch eine NURBS-Linienverlängerung definiert wird;
- Festlegen (1004) der Länge jeder NURBS-Linienverlängerung erstens auf einen Wert der geometrischen Höhe abzüglich der zentralen Koaptationshöhe jedes Neo-Klappensegels für die Mittelachse eines jeweiligen Neo-Klappensegels und zweitens auf einen Wert der zwei komplementären Radialachsen für die dazwischenliegenden geometrischen Positionen zwischen der Mittelachse beiderseits davon und für die untere Kante der 2D-verflachten Koaptationsfläche;
- Modellieren (1006) der interpolierten Neo-LIL-Kurve mit den unteren kommissuralen Landmarken sowie Landmarken, die sich an den Enden jeder NURBS-Linienverlängerung befinden; und
- Konstruieren (1008) von lasttragenden 2D-Neo-Flächen als Netzwerk von Neo-ILC-Kurven, Neo-LIL-Kurven und Neo-gH-Kurven.

9. Verfahren nach Anspruch 8, wobei mehr Achsen, die allen auf den LIL-Kurven platzierten Landmarken entsprechen, verwendet werden, um die Zuverlässigkeit der 2D-Verflachung der lasttragenden Fläche zu erhöhen.

10. Verfahren nach einem der Ansprüche 1 bis 9, wobei der Schritt (114) des Erzeugens des 3D-Modells ferner einen Schritt eines Exportierens des 3D-Modells als Mesh-Datei für einen 3D-Druck oder eine stereoskopische 3D-Visualisierung umfasst.

11. Verfahren nach einem der Ansprüche 1 bis 10, das ferner einen Schritt eines Druckens der patientenspezifischen Schablonen aller Aortenklappensegel zur Verwendung als autologe Perikard-Schneidhilfe für eine Aortenklappen-Neokuspidisierungsoperation umfasst.

12. System zum Erzeugen von patientenspezifischen Schablonen von Aortenklappensegeln, das Mittel zum Durchführen der Schritte des Verfahrens nach einem der Ansprüche 1 bis 11 aufweist.

13. Computerprogrammprodukt, wobei das Computerprogrammprodukt Codeanweisungen zum Durchführen der Schritte des Verfahrens nach einem der Ansprüche 1 bis 11 bei Ausführung durch einen Computer aufweist.

## Revendications

1. Procédé mis en œuvre par ordinateur (100) permettant de générer des modèles de feuillets aortiques spécifiques au patient, comprenant :
- génération (104) d'un modèle 3D à partir d'images numériques (102) d'une racine aortique native et de feuillets aortiques d'un patient ;
- génération (106) de modèles de néo-feuillets 3D par simulation in silico de la néocuspidisation des feuillets aortiques à partir du modèle 3D avec des paramètres de surfaces de coaptation développables et de surfaces porteuses non développables des feuillets aortiques ;
- réalisation ( 108) d'un aplatissement de surface 2D des surfaces de coaptation des modèles de néo-feuillets 3D ;
- réalisation (110) d'un aplatissement de surface 2D des surfaces porteuses des modèles de néo-feuillets 3D ;
- fusion (112) d'une surface de coaptation 2D avec une surface porteuse 2D pour chaque modèle de néo-feuillet ; et
- génération (114) d'un modèle spécifique au patient pour chaque feuillet aortique.

2. Procédé selon la revendication 1, dans lequel l'étape (104) de génération d'un modèle 3D comprend une première étape de traitement (202) basée sur une approche de morphométrie géométrique "GM" et une deuxième étape de traitement (204) basée sur des courbes de splines de base rationnelles non uniformes "NURBS".

3. Procédé selon la revendication 2, dans lequel la première étape de traitement (202) basée sur la morphométrie géométrique comprend :
- en utilisant les valeurs de seuil Hounsfield, segmenter (302) la racine aortique en plaçant des repères de coaptation commissurale supérieure et inférieure et des repères correspondant au nadir de chaque sinus ;
- en utilisant la reconstruction multiplanaire, placer (304) des repères correspondant aux points de coaptation centraux supérieurs et inférieurs, et placer des semi-repères le long des marges libres des feuillets (LFMs) et des lignes inférieures de coaptation (ILCs) ; et
- en utilisant le rendu de surface, placer (306) des semi-repères le long de la ligne d'insertion de feuillet (LIL) sur la racine aortique.

4. Procédé selon la revendication 2 ou 3, dans lequel la deuxième étape de traitement (204) basée sur des courbes NURBS comprend :
- en utilisant le point de contrôle des courbes NURBS, tracer (502) chaque déterminant géométrique des feuillets et des hauteurs de coaptation centrale, latérale et commissurale ;
- en utilisant les déterminants géométriques, modéliser (504) une surface NURBS 3D de coaptation et modéliser une surface NURBS 3D porteuse ; et
- fusionner (506) une surface NURBS 3D de coaptation avec une surface NURBS 3D porteuse pour générer une modélisation de surface 3D pour chaque feuillet aortique.

5. Procédé selon l'une quelconque des revendications 1 à 4, dans lequel l'étape (106) de génération de modèles de néo-feuillets 3D comprend (804) l'utilisation d'un centre de coaptation natif dans la simulation de l'extension de feuillets (804).

6. Procédé selon l'une quelconque des revendications 1 à 4, dans lequel l'étape (106) de génération de modèles de néo-feuillets 3D comprend (806) l'utilisation de centroïdes de la jonction sino-tubulaire et de la base de racine aortique dans la simulation de résections de feuillets.

7. Procédé selon l'une quelconque des revendications 1 à 6, dans lequel l'étape (108) de réalisation d'un aplatissement de surface 2D des surfaces de coaptation est effectuée par l'intermédiaire d'une commande logicielle standard d'un outil de conception assistée par ordinateur, les surfaces de coaptation étant des surfaces développables présentant une courbure dans une seule direction.

8. Procédé selon l'une quelconque des revendications 1 à 7, dans lequel l'étape (110) de réalisation d'un aplatissement de surface 2D des surfaces porteuses est effectuée par un algorithme dédié, les surfaces porteuses étant des surfaces non développables, comprenant les étapes suivantes :
- modélisation (1002) des surfaces porteuses dans le même plan que celui utilisé pour l'aplatissement de surface 2D des surfaces de coaptation, en étendant les surfaces porteuses à l'aide de lignes NURBS définissant ainsi l'extension de lignes NURBS ;
- ajustement (1004) de la longueur de chaque extension de lignes NURBS, premièrement, à une valeur de la hauteur géométrique moins la hauteur de coaptation centrale de chaque néo-feuillet pour l'axe médian d'un néo-feuillet considéré, et, deuxièmement, à une valeur des deux axes radiaux complémentaires pour les positions géométriques intermédiaires entre l'axe médian, de part et d'autre de celui-ci, et pour le bord inférieur de la surface de coaptation 2D aplatie ;
- modélisation (1006) de la courbe néo-LIL interpolée avec les repères commissuraux inférieurs plus les repères situés aux extrémités de chaque extension de lignes NURBS ; et
- construction (1008) de néo-surfaces porteuses 2D sous forme de réseau de courbes néo-ILC, de courbes néo-LIL et de courbes néo-gH.

9. Procédé selon la revendication 8, dans lequel davantage d'axes correspondant à chaque repère placé sur les courbes LIL, sont utilisés pour accroître la fiabilité de l'aplatissement de surface 2D de la surface porteuse.

10. Procédé selon l'une quelconque des revendications 1 à 9, dans lequel l'étape (114) de génération du modèle 3D comprend en outre une étape d'exportation du modèle 3D sous forme d'un fichier maillé pour une impression 3D ou pour une visualisation stéréoscopique 3D.

11. Procédé selon l'une quelconque des revendications 1 à 10, comprenant en outre une étape d'impression du modèle spécifique au patient de chaque feuillet aortique à utiliser comme guide de coupe péricardique autologue pour une chirurgie de néocuspidisation de la valve aortique.

12. Système de génération de modèles de feuillets aortiques spécifiques au patient comprenant des moyens pour exécuter les étapes du procédé selon l'une quelconque des revendications 1 à 11.

13. Produit de programme informatique, ledit produit de programme informatique comprenant des instructions de code pour exécuter, lorsqu'il est exécuté par un ordinateur, les étapes du procédé selon l'une quelconque des revendications 1 à 11.
